# DEMANDE DE BREVET EUROPEEN

(11) **EP 1 064 941 A1**
(43) Date de publication de la demande: **03.01.2001**
(21) Numéro de dépôt: 00401781.0
(22) Date de dépôt: 22.06.2000
(51) Int. Cl.: A61K 31/381, A61P 1/00, A61P 1/04, A61P 1/06, A61P 1/12, A61P 1/14, A61K 31/4045

(54) **Utilisation de ligands mélatoninergiques pour l'obtension de compositions pharmaceutiques destinées à la prévention ou au traitement des pathologies du système gastrointestinal**

(30) Priorité: 23.06.1999 FR 9908019
(71) Demandeur: ADIR ET COMPAGNIE, 92415 Courbevoie Cédex (FR)
(72) Inventeur: Pellissier, Sonia, 01300 Nattages (FR); Merle, Anne, 73000 Chambery (FR); Delagrange, Philippe, 92130 Issy Les Moulineaux (FR); Bennejean, Caroline, 94220 Charenton le Pont (FR); Renard, Pierre, 78150 Le Chesnay (FR)

(57) **Abrégé**

Utilisation de la mélatonine et des ligands mélatoninergiques pour l'obtention de compositions pharmaceutiques destinées a la prévention et/ou au traitement des pathologies du système gastrointestinal

## Description

L'invention concerne l'utilisation de la mélatonine et des ligands mélatoninergiques pour l'obtention de compositions pharmaceutiques destinées à la prévention ou au traitement des pathologies du système gastrointestinal et des troubles de la motricité associés.

Chez l'homme ou chez l'animal, l'activité myoélectrique des cellules musculaires lisses de la paroi de l'intestin grêle est constituée de deux types de variations de potentiels :
- des ondes lentes permanentes
- des ondes rapides intermittentes ou salves de potentiels se superposant aux ondes lentes et qui sont seules responsables des contractions de la paroi intestinale.

Le profil moteur de l'intestin évolue en fonction de l'état digestif. En période préprandiale, il se présente sous la forme de complexes myoélectriques cycliques se propageant de la partie proximale (duodénum) à la partie distale (iléon) de l'intestin.

L'ingestion de nourriture entraîne l'interruption immédiate de l'organisation de la motricité intestinale en complexes myoélectriques au profit d'une activité irrégulière postprandiale persistant plusieurs heures. La durée de cette réponse postprandiale est directement liée à la quantité de nourriture ingérée, à sa consistance (liquide ou solide) et à sa composition biochimique.

Il existe par ailleurs un rythme nycthéméral au niveau de la motricité intestinale pré et postprandiale. La durée de l'activité irrégulière postprandiale est raccourcie en période nocturne en comparaison d'une réponse postprandiale induite par un repas identique durant la phase diurne.

Les troubles fonctionnels intestinaux sont en général dus à une perturbation de la motricité intestinale. Ils doivent être considérés comme une série de perturbations du contrôle neuro-hormonal du tube digestif qui entraîne au premier chef des modifications de l'activité motrice pouvant induire des troubles de la sécrétion intestinale ainsi qu'un état d'hypersensibilité. Ces perturbations sont accentuées par le stress ou d'autres troubles d'origine psychologique.

Parmi les troubles digestifs ayant pour cause ou pour conséquence un dysfonctionnement de la motricité intestinale on peut citer, entre autres, le syndrome de l'intestin irritable, la constipation chronique idiopathique, les troubles moteurs digestifs liés à la maladie inflammatoire de l'intestin, ainsi que les pathologies du tube digestif entraînant des troubles de la motricité intestinale.

Le syndrome de l'intestin irritable est un élément fréquent de l'ensemble des troubles fonctionnels du tube digestif. Il se manifeste cliniquement par des désordres gastrointestinaux très hétérogènes. Ce syndrome est caractérisé par des douleurs abdominales fréquentes, s'accompagnant de constipation ou de diarrhée résultant d'une motricité anormale de l'intestin en périodes pré et postprandiales :
- en période préprandiale, les patients présentent des troubles de la périodicité des complexes myoélectriques migrants au cours du nycthémère. En effet, alors que chez les sujets sains, la période des complexes myoélectriques est plus longue le jour que la nuit, chez les patients, la période des complexes myoélectriques est aussi courte le jour que la nuit.
   Ainsi le syndrome de l'intestin irritable se traduit par la disparition de la variation nycthémérale de la fréquence des complexes (Phillips S.F., Motor patterns in the irritable bowel and pseudo-obstruction in Pharmacotherapy of gastrointestinal motor disorders. Edit. J. Dent Reed Healthcare Communications Sydney 199, pp 57-66) et la motricité de l'iléon semble être plus particulièrement le siège d'une activité irrégulière intense et rapidement propagée à l'origine de crampes intestinales très violentes.
- en période postprandiale, Evans et al. (Gastroparesis ans small bowel dysmotility in irritable bowel syndrome. Dig. Dis. Sci., 1997, 42, pp 2087-2093) ont décrit la présence anormale de complexes myoélectriques préprandiaux survenant au sein de l'activité irrégulière postprandiale.
   D'une façon plus générale, dans ce syndrome l'intestin répond souvent de manière exagérée à certains stimuli comme le repas et les hormones telle que la cholécystokinine.

Ces observations montrent clairement que le traitement des symptômes digestifs liés au syndrome de l'intestin irritable passe par une régularisation de la motricité préprandiale et postprandiale.

La constipation chronique idiopathique se manifeste par une fréquence de selles inférieure à une par semaine. Ses causes physiopathologiques sont inconnues et les traitements classiques préconisant une alimentation riche en fibres et l'utilisation de laxatifs sont généralement inefficaces et de plus en plus contestés dans leur principe. Cette affection touche préférentiellement les femmes et débute le plus souvent au moment de l'adolescence. Ces patientes présentent un temps de transit colique fortement ralenti. Il a été montré que dans les formes les plus sévères de constipation les troubles de la motricité ne se limitent pas au côlon mais s'étendent à l'intestin grêle et à l'estomac. Ainsi, il a récemment été mis en évidence que les complexes myoélectriques présentent des anomalies durant les phases diurne et nocturne (Panagamuwa B. et al, Motor abnormalities in rhe terminal ileum of patients with chronic idiopathic constipation. Br. J. Surg., 1994, 81, pp 1685-1688).

Les maladies inflammatoires de l'intestin sont souvent des pathologies chroniques évoluant par poussées entrecoupées de périodes de rémission. Elles sont caractérisées par un état d'hyperactivation du système immunitaire de l'intestin dont l'origine est inconnue. Les trois principales maladies inflammatoires chroniques de l'intestin sont la rectocolite hémorragique, la maladie de Crohn et les colites. La rectocolite hémorragique affecte essentiellement le colon et est associée à des diarrhées sévères. L'intestin grêle n'est pas atteint à l'exception d'une portion de l'iléon terminal. La maladie de Crohn peut atteindre tous les segments du tube digestif bien que le siège le plus fréquent de l'inflammation soit l'iléon terminal et l'iléo-colon. Les colites affectent essentiellement le colon. Leurs origines sont souvent indéterminées et les symptômes aigus ou chroniques peuvent être amplifiés ou déclenchés par des situations de stress.
Dans ces maladies, le processus inflammatoire n'affecte pas uniquement la muqueuse intestinale mais également les couches les plus profondes de la paroi incluant les neurones et les cellules musculaires lisses. De nombreuses études réalisées chez l'animal et chez l'homme indiquent que ce syndrome inflammatoire s'accompagne de perturbations de la motricité intestinale, non seulement au niveau du segment enflammé mais également au niveau des territoires sains. Ainsi des modifications de la vidange gastrique et du temps de transit oro-caecal ont été observées chez des patients souffrant de colites ulcéreuses. Dans les colites expérimentales induites chez le rat, il a été montré que la motricité de l'iléon est aussi perturbée et que la fréquence des complexes myoélectriques de l'iléon est anormalement élevée en raison d'un raccourcissement de la phase de repos (Aube A.C. et al., Altered myoelectrical activity in noninflamed ileum of rats with colitis induced by trinitrobenzenz sulphonic acid. Neurogastroenterol. Motility, 1999, 11, pp 55-62).
Cependant, il semble que le sens de variation de la fréquence des complexes myoélectriques iléaux dépend de la localisation initiale de l'inflammation : Dwinell et al. (Dwinell M.B., Intestinal myoelectricalalterations in rats chronically infected with the tapeworm *hymenolepis diminuta.* Am. J. Physiol., 1994, 267, ppG851-858) rapportent que les inflammations de l'iléon s'accompagnent d'une réduction importante de la fréquence de leurs complexes myoélectriques par allongement de l'activité irrégulière alors que les inflammations du colon entraînent une augmentation de la fréquence des complexes myoélectriques iléaux.

Selon l'origine de l'inflammation il faudrait soit supprimer l'augmentation de la fréquence des complexes myoélectriques soit augmenter la fréquence pour supprimer les symptômes digestifs associés au dysfonctionnement de la motricité iléale.

D'autres syndromes inflammatoires tels que les allergies alimentaires entraînent des perturbations de la motricité intestinale. Expérimentalement, une toxine bactérienne peut entraîner la disparition des complexes myoélectriques de l'intestin en trois jours chez un animal non sensibilisé et dans les premières heures qui suivent le contact avec l'allergène chez un animal sensibilisé. Ces complexes myoélectriques sont remplacés par une activité irrégulière rapidement propagée responsable d'une accélération du transit et d'une diarrhée.

Des études récentes rapportent qu'une des principales complications digestives de la cirrhose du foie et de l'insuffisance hépatique est la grande prévalence de surinfections bactériennes liée au sur-développement de la flore intestinale (Madrid A.M. et al., Altered small bowel motility in patients with liver cirrhosis deppends on severity of liver disease. Dig. Dis. Sci., 1997, 42, pp 738-742) qui peut entraîner l'apparition de péritonites spontanées récurrentes (Chang C.S. et al., Small intestine dysmotility and bacterial overgrowth in cirrhotic patients with spontaneous bacterial peritonitis. Hepatology, 1998, 28, pp 1187-1190).
L'exploration fonctionnelle de la motricité digestive chez ces patients révèle des anomalies du complexe myoélectrique migrant de l'intestin grêle. Dans la majorité des cas les complexes se raréfient et peuvent totalement disparaître. De plus, les rares complexes qui persistent se propagent à une vitesse très ralentie.
Il a été démontré qu'un tel profil moteur intestinal favorise la stagnation de l'eau et des sécrétions digestives dans la lumière intestinale et que ceci est à l'origine de la pullulation de l'écosystème microbien de l'intestin.

En conséquence, il paraît important de normaliser la motricité intestinale lorsque celle-ci est perturbée ce qui est le cas dans la majorité des pathologies du système digestif.

L'utilisation d'un agent pharmacologique qui permettrait la restauration d'une fréquence physiologique des complexes myoélectriques, permettrait d'éviter ce genre de complications.

En particulier, toute molécule active sur les complexes myoélectriques (induction ou inhibition en fonction de la pathologie) sera utile dans le traitement des pathologies mentionnées ci-dessus. Il en sera de même pour des molécules capables de normaliser la durée des profils moteurs en période pré ou postprandiale.

La demanderesse a ainsi découvert que la mélatonine et les ligands mélatoninergiques, agonistes ou antagonistes, étaient utiles dans la prévention et/ou dans le traitement des pathologies du système gastrointestinal.

Il a en effet été montré par des études *in vivo* que les ligands mélatoninergiques, agonistes ou antagonistes, sont capables de moduler la motricité intestinale en intervenant au niveau des complexes myoélectriques et/ou sur la durée de la réponse postprandiale.

En particulier, la mélatonine et les ligands mélatoninergiques, agonistes ou antagonistes sont utiles dans la prévention et/ou dans le traitement du syndrome de l'intestin irritable, de la constipation idiopathique, des troubles moteurs digestifs liés à la maladie inflammatoire de l'intestin, ainsi que des pathologies du tube digestif entraînant des troubles de la motricité intestinale.

L'invention concerne donc l'utilisation de la mélatonine et des ligands mélatoninergiques, agonistes ou antagonistes, pour l'obtention de compositions pharmaceutiques destinées à la prévention et/ou au traitement des pathologies du système gastrointestinal.

Plus particulièrement, l'invention concerne l'utilisation, pour l'obtention de compositions pharmaceutiques destinées à la prévention et/ou au traitement des pathologies du système gastrointestinal, de la mélatonine et des ligands mélatoninergiques comme par exemple les ligands :
- de formule (I) 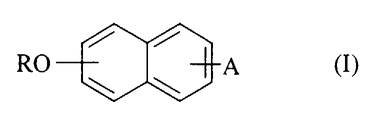 tels que décrits dans la demande de brevet EP447285,
- de formule (II) 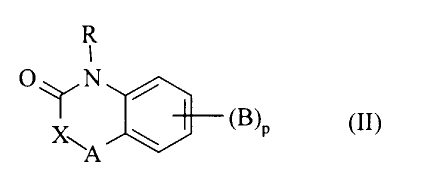 tels que décrits dans la demande de brevet EP506539,
- de formule (III) 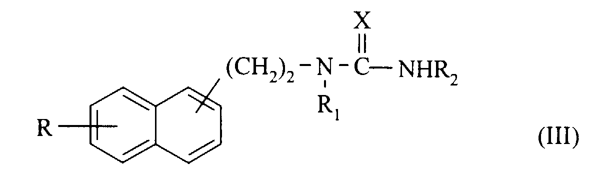 tels que décrits dans la demande de brevet EP530087,
- de formule (IV) 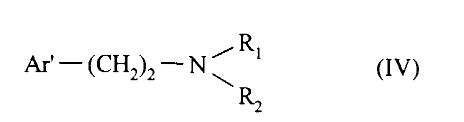 tels que décrits dans la demande de brevet EP527687,
- de formule (V) 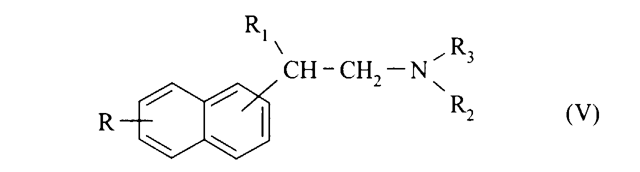 tels que décrits dans la demande de brevet EP562956,
- de formule (VI) 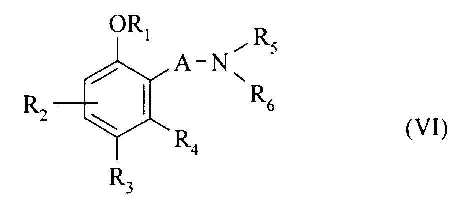 tels que décrits dans la demande de brevet EP591057,
- de formule (VII) 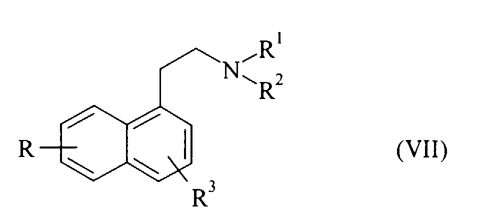 tels que décrits dans la demande de brevet EP662471,
- de formule (VIII) 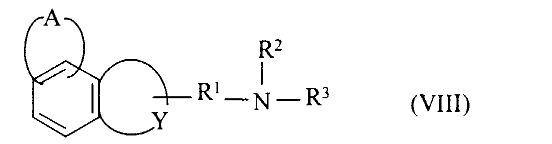 tels que décrits dans la demande de brevet EP708099,
- de formule (IX) 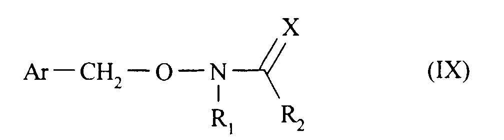 tels que décrits dans la demande de brevet EP709371,
- de formule (X) 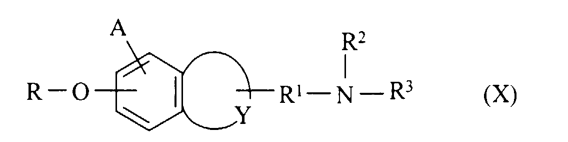 tels que décrits dans la demande de brevet EP745583,
- de formule (XI) 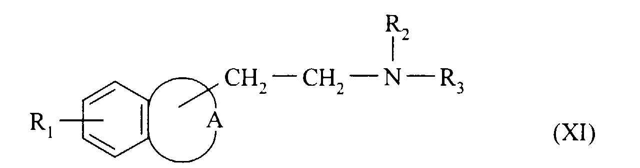 tels que décrits dans la demande de brevet EP721938,
- de formule (XII) 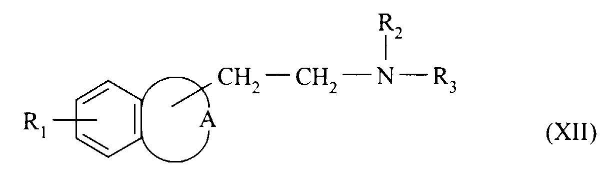 tels que décrits dans la demande de brevet EP721947,
- de formule (XIII) 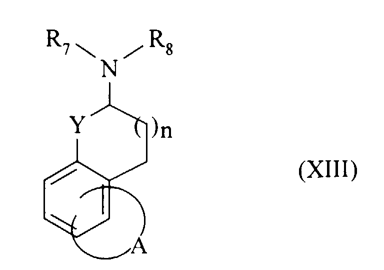 tels que décrits dans la demande de brevet EP737670,
- de formule (XIV) 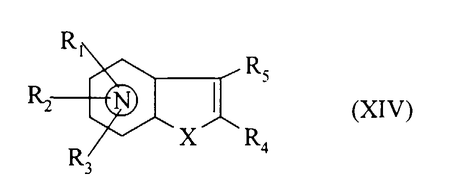 tels que décrits dans la demande de brevet EP737685,
- de formule (XV) 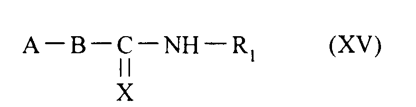 tels que décrits dans la demande de brevet EP745584,
- de formule (XVI) 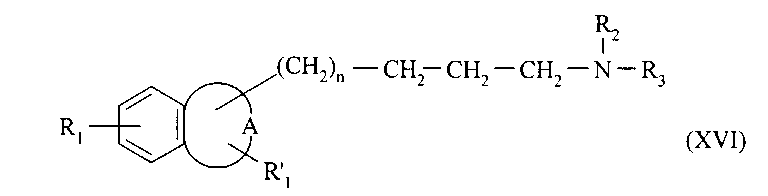 tels que décrits dans la demande de brevet EP745586,
- de formule (XVII) 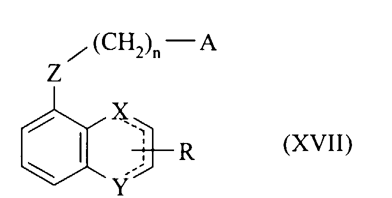 tels que décrits dans la demande de brevet EP873993,
- de formule (XVIII) 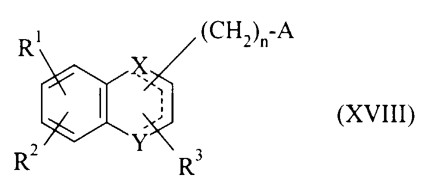 tels que décrits dans la demande de brevet WO98/52935,
- de formule (XIX) 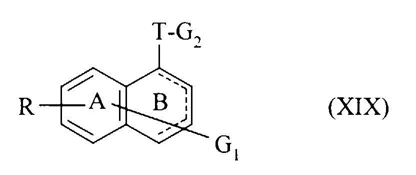 tels que décrits dans la demande de brevet EP919541.
- de formule (XX) 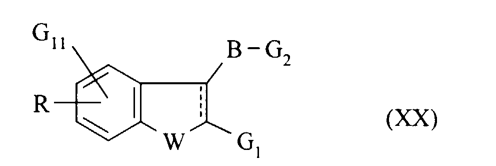 tels que décrits dans la demande de brevet EP926145,
- de formule (XXI)

   R - A - R' (XXI)

   tels que décrits dans les demandes de brevet WO9958495 et WO9958496,
- de formule (XXII) 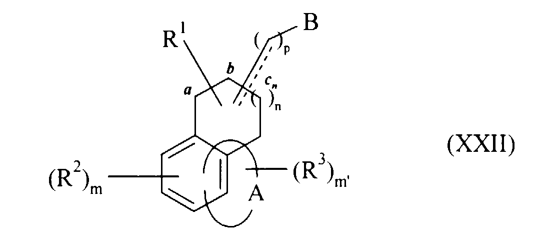 tels que décrits dans la demande de brevet WO9936392,
- ou de formule (XXIII) 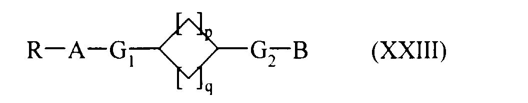 tels que décrits dans la demande de brevet EP0994102.

L'invention concerne également l'utilisation, pour l'obtention de compositions pharmaceutiques destinées à la prévention et/ou au traitement des pathologies du système gastrointestinal, d'autres ligands mélatoninergiques que ceux représentés par les formules (I) à (XIX) comme par exemple les composés décrits dans les demandes WO 9838991, EP 747346, WO 9825606, EP 706994, EP 747345, EP 745597, WO 9738682, WO 9529173, WO 9517405, WO 9743272, WO 9705098, WO 9608466, WO 9732871, WO 9706140, WO 9527712, EP 281242 ou EP 655243.

Un aspect avantageux de l'invention concerne l'utilisation, pour l'obtention de compositions pharmaceutiques destinées à la prévention et/ou au traitement des pathologies du système gastrointestinal, de ligands mélatoninergiques agonistes. Plus particulièrement, l'invention concerne l'utilisation, pour l'obtention de compositions pharmaceutiques destinées à la prévention et/ou traitement des pathologies du système gastrointestinal, des ligands mélatoninergiques de formule (I), (VIII) ou (XVII) comme par exemple le N-[2-(7-méthoxy-1-naphtyl)éthyl]acétamide, le N-[2-(8,9-dihydro-7*H*-furo[3,2-f]chromèn-1-yl)éthyl]acétamide et le 4-(2,3-dihydro-1,4-benzodioxin-5-yl)-N-méthylbutanamide.

Un autre aspect avantageux de l'invention concerne l'utilisation, pour l'obtention de compositions pharmaceutiques destinées à la prévention et/ou au traitement des pathologies du système gastrointestinal, de ligands mélatoninergiques antagonistes.

De façon préférentielle, l'invention concerne l'utilisation, pour l'obtention de compositions pharmaceutiques destinées à la prévention et/ou au traitement des pathologies du système gastrointestinal, des ligands mélatoninergiques de formule (XI) et encore plus particulièrement de formule (XI_{A)}, cas particulier des composés de formule (XI) :

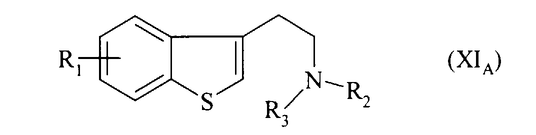

Encore plus préférentiellement, l'invention concerne l'utilisation, pour l'obtention de compositions pharmaceutiques destinées à la prévention et/ou au traitement des pathologies du système gastrointestinal, du N-[2-(5-éthyl-1-benzothiophèn-3-yl)éthyl]acétamide.

L'invention s'étend également aux compositions pharmaceutiques contenant de la mélatonine ou un ou plusieurs ligands mélatoninergiques, agonistes ou antagonistes, en combinaison avec un ou plusieurs excipients pharmaceutiquement acceptables, utile dans la prévention et/ou dans le traitement des pathologies du système gastrointestinal.

L'invention s'étend également aux compositions pharmaceutiques contenant de la mélatonine ou un ou plusieurs ligands mélatoninergiques, agonistes ou antagonistes, de formules (I) à (XXIII) telles que définies précédemment en combinaison avec un ou plusieurs excipients pharmaceutiquement acceptables, utile dans la prévention et/ou dans le traitement des pathologies du système gastrointestinal.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou plusieurs prises.

Les exemples suivants illustrent l'invention mais ne la limite en aucune façon et attestent de l'activité *in vivo* de la mélatonine et des ligands mélatoninergiques.

### EXEMPLE A : Effets de ligands mélatoninergiques sur les complexes myoélectriques intestinaux chez le rat.

Des rats mâles Wistar adultes placés en cycle lumière/obscurité 12h/12h sont implantés sous anesthésie d'électrodes sur la paroi de l'intestin afin d'enregistrer l'activité myoélectrique. Les rats ont un accès limité (30 min) à la nourriture pendant la période diurne.

Les composés de l'invention agonistes mélatoninergiques ou la mélatonine sont administrés par voie intraveineuse à la dose de 1 mg/kg, 10 min avant le repas. Les agonistes mélatoninergiques ou la mélatonine induisent l'apparition de complexes myoélectriques pendant la réponse motrice postprandiale. Ces complexes myoélectriques apparaissent tout d'abord sur le duodénum avec un délai de 50 min puis ils se propagent sur le jéjunum et l'iléon.

L'induction des complexes myoélectriques est totalement bloquée par un prétraitement par les antagonistes mélatoninergiques de l'invention : par exemple le N-[2-(5-éthyl-1-benzothiophèn-3-yl)éthyl]acétamide à la dose de 2 mg/kg en sous-cutanée administré 30 min avant l'agoniste empêche toute formation de complexe myoélectrique.

Ces résultats montrent :
- que les composés agonistes de l'invention jouent sur les mécanismes de création des complexes myoélectriques
- que les antagonistes de l'invention peuvent interrompre ces complexes.

### EXEMPLE B: Effets de ligands mélatoninergiques sur la durée de la réponse postprandiale chez le rat.

Chez le rat, la durée de la réponse postprandiale évaluée par l'activité motrice irrégulière intestinale, est plus courte en période d'obscurité que pendant la période diurne et ceci pour une même quantité d'aliments ingérés. Afin de confirmer que cette diminution de la durée pouvait être due à la sécrétion nocturne de mélatonine, le protocole suivant a été réalisé : des rats mâles Wistar adultes habitués à un cycle lumière/obscurité 12h/12h sont implantés sous anesthésie d'électrodes sur la paroi de l'intestin afin d'enregistrer l'activité myoélectrique. Les rats ont un accès limité (30 min) à la nourriture soit pendant la période nocturne soit pendant la période diurne. Les molécules de l'invention sont administrées 30 min avant l'accès à la nourriture. Le paramètre évalués dans ces études est la durée de l'activité irrégulière motrice. Les périodes de complexes myoélectriques ne sont pas prises en compte dans le cas d'induction par la molécule testée.

La durée de la réponse postprandiale nocturne est allongée par un prétraitement par les antagonistes mélatoninergiques de l'invention. Par exemple, le N-[2-(5-éthyl-1-benzothiophèn-3-yl)éthyl]acétamide administré 5h après l'apparition de l'obscurité entraîne un allongement de la réponse postprandiale qui devient d'une durée équivalente à la réponse observée le jour.

La durée de la réponse postprandiale diurne est diminuée par un prétraitement par les agonistes mélatoninergiques de l'invention ou la mélatonine. Par exemple, la mélatonine (100 µg/kg, intraveineuse) administrée 4h après l'apparition de la lumière réduit la durée de la réponse postprandiale et la ramène à une durée de type nocturne.

Ces résultats montrent que les composés de l'invention agonistes ou antagonistes mélatoninergiques peuvent moduler la durée de la réponse postprandiale.

### EXEMPLE C : Effets de ligands mélatoninergiques sur un modèle d'inflammation intestinale induite par l'indométhacine

L'inflammation intestinale induite par administration d'indométhacine chez le rat est considérée comme un modèle animal physiopathologique présentant des modifications du comportement et de la motricité intestinale similaires à celles observées chez les patients atteints de la maladie de Crohn (Yamada T. et al, 1993, Mechanisms of acute and chronic intestinal inflammation induces by indomethacin, Inflammation, 17-6, 641-62 ; Gurbuz V. et al, 1999, Role of nitric oxide in indomethacin-induced gastric mucosal dysfunction in the rat, Experimental Physiology, 84, 319-332 ; Chen K. et al, 1999, Expression of NO II and its role in the experimental small bowel ulceration in rats, Surgery, 126, 553-561).

Des rats mâles Wistar adultes placés en cycle lumière/obscurité 12h/12h sont implantés sous anesthésie d'électrodes sur la paroi de l'intestin afin d'enregistrer l'activité myoélectrique. Les animaux sont divisés en plusieurs groupes.

Le groupe de rats témoins reçoit deux administrations d'indométhacine (7,5 mg/kg sc) espacées de 24 h. Les autres groupes de rats sont prétraités par la mélatonine ou un ligand mélatoninergique (50 mg/kg ip) dix minutes avant les deux administrations d'indométhacine.

Les animaux sont suivis sur le plan comportemental (prise alimentaire), physiologique (aspect du pelage, diarrhées) et motricité intestinale.

Tous les rats traités à l'indométhacine présentent une anorexie, une perte de poils, des diarrhées et une diminution de la fréquence des complexes myoélectriques au niveau du duodénum, du jéjunum et de l'iléon. Les rats traités par la mélatonine ou un ligand mélatoninergique ne présentent plus de diarrhées, de perte de poils, de diminution de la prise alimentaire et de la motricité intestinale.

Ces résultats montrent que les composés de l'invention ou la mélatonine préviennent tous les symptômes conséquents d'une inflammation intestinale.

### Conclusion

La mélatonine et les ligands mélatoninergiques, agonistes ou antagonistes, permettent de modifier l'activité motrice intestinale tant au niveau des activités organisées en complexes myoélectriques qu'au niveau de l'activité irrégulière. Ces deux types d'activité étant altérées dans les pathologies du système gastrointestinal, ces molécules permettent donc au clinicien d'agir contre les troubles de la motricité intestinale.

## Revendications

1. Utilisation de la mélatonine et des ligands mélatoninergiques pour l'obtention de compositions pharmaceutiques destinées à la prévention et/ou au traitement des pathologies du système gastrointestinal.

2. Utilisation selon la revendication 1 de la mélatonine pour l'obtention de compositions pharmaceutiques destinées à la prévention et/ou au traitement des pathologies du système gastrointestinal.

3. Utilisation selon la revendication 1 des ligands mélatoninergiques pour l'obtention de compositions pharmaceutiques destinées à la prévention et/ou au traitement des pathologies du système gastrointestinal.

4. Utilisation selon la revendication 1 des ligands mélatoninergiques agonistes pour l'obtention de compositions pharmaceutiques destinées à la prévention et/ou au traitement des pathologies du système gastrointestinal.

5. Utilisation selon la revendication 1 des ligands mélatoninergiques antagonistes pour l'obtention de compositions pharmaceutiques destinées à la prévention et/ou au traitement des pathologies du système gastrointestinal.

6. Utilisation selon la revendication 1 du N-[2-(5-éthyl-1-benzothiophèn-3-yl)éthyl]acétamide pour l'obtention de compositions pharmaceutiques destinées à la prévention et/ou au traitement des pathologies du système gastrointestinal.

7. Compositions pharmaceutiques contenant de la mélatonine ou un ou plusieurs ligands mélatoninergiques en combinaison avec un ou plusieurs excipients pharmaceutiquement acceptables utiles dans la prévention et/ou dans le traitement des pathologies du système gastrointestinal.
